# EUROPEAN PATENT APPLICATION

(11) **EP 2 786 697 A1**
(43) Date of publication of application: **08.10.2014**
(21) Application number: 12852843.7
(22) Date of filing: 08.03.2012
(51) Int. Cl.: A61B 3/10, A61B 3/12, A61B 3/14

(54) **MICROSCOPE FOR MONITORING OPTICAL COHERENCE TOMOGRAPHY**

(30) Priority: 01.12.2011 KR 20110127595; 06.03.2012 KR 20120022818
(71) Applicant: Kyungpook National University Industry-Academic Cooperation Foundation, Daegu 702-701 (KR)
(72) Inventor: KIM, Jee Hyun, Daegu 705-023 (KR); KIM, Hong Kyun, Daegu 706-031 (KR); LEE, Chang Ho, Daegu 705-030 (KR); KIM, Kyung Un, Daegu 701-210 (KR); LEE, Jun Hun, Daegu 706-041 (KR); HAN, Seung Hoon, Gumi-si Gyeongsangbuk-do 730-310 (KR); PARK, Ki Beom, Daegu 706-160 (KR)
(74) Representative: Scholz, Volker
(86) International application number: PCT/KR2012/001704
(87) International publication number: WO 2013/081252

(57) **Abstract**

Provided is a microscope for monitoring optical coherence tomography. The microscope for monitoring optical coherence tomography includes a microscope image providing unit which is configured to include a first objective lens, a beam splitter, and an ocular lens which are sequentially disposed from the front of a sample mount and an optical coherent tomography (OCT) image providing unit which generates an OCT image and provides the OCT image to the beam splitter of the microscope image providing unit, wherein the OCT image providing unit generates the OCT image of the same position as the surface image of the sample and provides the OCT image to the beam splitter of the microscope image providing unit, and in the case where the OCT image is provided through the beam splitter to the OCT image providing unit, the microscope image providing unit simultaneously outputs the surface image and the OCT image of the sample to the ocular lens.

## Description

### [Technical Field]

The present invention relates to a microscope for monitoring optical coherence tomography, and more particularly, to a microscope for monitoring optical coherence tomography where an optical coherent tomography (OCT) image is provided to the microscope, so that a microscope image and a biological tomography image can be simultaneously output to an external portion of the microscope.

### [Background Art]

In general, eyes are organs which sense intensity and wavelength of light. The organs have functions of sensing brightness, identifying the direction of light, recognizing images of objects, and so on. The eyeballs are contained in a pair of left and right eye orbits at the front side of skull. The eyes are protected by upper and lower eye lids. The eyeballs are connected to optic nerve. The wall of eyeball has three layers, and the outmost layer is configured with cornea and sclera. The cornea is sometimes called a black iris.

These days, due to change of the environment, various surgeries have been practiced with respect to eyes. For example, surgery for eye disease such as cataracts, dry eye, and glaucoma, LASEK or LASIK eye surgery for vision correction, eximer laser surgery, ICL surgery, and the like have been practiced. In many cases, surgical microscopes are used during surgery.

The surgical microscope is configured include a light source which illuminates light on a diseased part of a patient by using an electric power source, an object lens which widens an image of an eyeball of the patient, and an ocular lens which allows the widened image of the eyeball to be visually perceived. In addition, in order that the state of the patient to be observed and the surgery can be practiced without seeing the ocular lens, a beam splitter is disposed between the objective lens and the ocular lens, an image separated by the beam splitter is converted into an electrical signal by a CCD camera, the electrical signal is output to an monitor, so that the surgery can be easily practiced.

However, since the surgical microscope simply provides only the surface image of the eyeball, there is a problem that biological tissues cannot be easily identified due to a difference in brightness occurring in several layers of a biological body. In order to solve the problem, in general, after incision, surgical tools are inserted into the incised portion of the biological body for identification. In this case, there is a problem in that the biological body has inevitable cut, and the result of surgery is greatly affected by experience or a degree of expertness of the surgeon. Therefore, there is much difficulty in ensuring safety and accuracy of the surgery.

### [Disclosure]

### [Technical Problem]

In a microscope of the related art, only a surface image is provided through the microscope, and thus, biological tomography images are hard to identify, so that there is much difficulty in ensuring safety and accuracy of surgery. The present invention is to provide a microscope for monitoring optical coherence tomography (OCT) capable of outputting a microscope image and an OCT image to an external portion in order to solve the problem of the microscope of the related art.

### [Technical Solution]

According to an aspect of the present invention, there is provided a microscope for monitoring optical coherence tomography, including: a sample mount which a sample is mounted on; a microscope image providing unit which is configured to include a first objective lens and an ocular lens which are sequentially disposed from the front of the sample mount and provides a surface image of the sample by using first light generated by a first light source; and an OCT image providing unit which divides second light generated by a second light source into first and second light beams to apply the first and second light beams to a reference mirror and the sample, respectively, guides a path so as for the second light beam to be applied to the same position of the sample as the first light, generates an optical coherent tomography (OCT) image by using interference signals of a reference beam and a signal beam which are reflected from the reference mirror and the sample, respectively, and after that, provides the OCT image or an OCT image signal corresponding to the OCT image to the microscope image providing unit, wherein the OCT image providing unit generates the OCT image of the same position as the surface image of the sample and provides the OCT image to the microscope image providing unit.

In the microscope for monitoring optical coherence tomography according to the above aspect of the present invention, preferably, the microscope image providing unit is configured to further include a beam splitter between the first objective lens and the ocular lens, and in the case where the OCT image is provided from the OCT image providing unit to the beam splitter, the microscope image providing unit simultaneously outputs the surface image and the OCT image of the sample to the ocular lens.

In the microscope for monitoring optical coherence tomography according to the above aspect of the present invention, preferably, the OCT image providing unit is configured to include: a second light source which generates the second light having a predefined wavelength; an optical coupler which divides the second light into the first and second light beams to apply the first and second light beams to the reference mirror and the sample, respectively, and couples a reference beam and a signal beam which are reflected from the reference mirror and the sample, respectively; a reference path unit which guides a path so as for the first light beam to be applied to the reference mirror and provides the reference beam reflected from the reference mirror to the optical coupler; a sample path unit which guides a path so as for the second light beam to be applied to the same position of the sample as the first light and provides the signal beam reflected from the sample to the optical coupler; a photodetector which detects an interference signal of the reference beam and the signal beam which are coupled by the optical coupler; a signal processing unit which generates the OCT image by processing the interference signal detected by the photodetector; and an image output unit which outputs the OCT image generated by the signal processing unit to the beam splitter of the microscope image providing unit.

In the microscope for monitoring optical coherence tomography according to the above aspect of the present invention, preferably, the microscope image providing unit is configured to further include a display unit which is installed in a predetermined area to output an image on a screen according to an image signal provided from an external system, and in the case where an OCT image signal corresponding to the OCT image is provided from the OCT image providing unit to the display unit, the microscope image providing unit simultaneously outputs the surface image and the OCT image of the sample.

In the microscope for monitoring optical coherence tomography according to the above aspect of the present invention, preferably, the OCT image providing unit is configured to include: a second light source which generates the second light having a predefined wavelength; an optical coupler which divides the second light into the first and second light beams to apply the first and second light beams to the reference mirror and the sample, respectively, and couples a reference beam and a signal beam which are reflected from the reference mirror and the sample, respectively; a reference path unit which guides a path so as for the first light beam to be applied to the reference mirror and provides the reference beam reflected from the reference mirror to the optical coupler; a sample path unit which guides a path so as for the second light beam to be applied to the same position of the sample as the first light and provides the signal beam reflected from the sample to the optical coupler; a photodetector which detects an interference signal of the reference beam and the signal beam which are coupled by the optical coupler; a signal processing unit which generates the OCT image by processing the interference signal detected by the photodetector and outputs the OCT image signal corresponding to the generated OCT image; and a second communication module which provides the output OCT image signal to the display unit of the microscope image providing unit.

In the microscope for monitoring optical coherence tomography according to the above aspect of the present invention, preferably, the sample path unit is configured to include a dichroic mirror which is disposed between the first objective lens and the sample mount to reflect the second light beam having a predefined wavelength and to transmit light having other wavelengths, and the dichroic mirror guides a path so as for the second light beam to be applied to the same position of the sample as the first light.

In the microscope for monitoring optical coherence tomography according to the above aspect of the present invention, preferably, the sample path unit is configured to further include: a collimator which collimates the second light beam; an optical path changing unit which is disposed on the propagation path of the collimated second light beam to propagate the second light beam to the dichroic mirror and to provide the signal beam reflected again from the sample to the collimator and is driven to scan a surface of the sample; and a second objective lens which is disposed between the dichroic mirror and the optical path changing unit to allow the second light beam output from the optical path changing unit to be focused and to provide the second light beam to the dichroic mirror.

In the microscope for monitoring optical coherence tomography according to the above aspect of the present invention, preferably, the optical path changing unit is configured with any one of a Galvano scanner, and a polygon mirror, and an X-Y scanner.

In the microscope for monitoring optical coherence tomography according to the above aspect of the present invention, preferably, the image output unit is configured with any one of a beam projector, an LCD (Liquid Crystal Display), and an OLED (Organic Light-Emitting Display).

In the microscope for monitoring optical coherence tomography according to the above aspect of the present invention, preferably, in the case of a binocular microscope where the microscope image providing unit has two beam splitters and two ocular lenses, the OCT image providing unit provides the OCT image to at least one or more of the two beam splitters.

In the microscope for monitoring optical coherence tomography according to the above aspect of the present invention, preferably, the display unit is configured to include: a display panel which outputs the image on the screen according to the image signal; and a first communication module which receives the image signal from the external portion.

In the microscope for monitoring optical coherence tomography according to the above aspect of the present invention, preferably, the display panel is configured with any one of an LCD (Liquid Crystal Display), an OLED (Organic Light Emitting Diodes), and an LCoS (Liquid Crystal on Silicon).

In the microscope for monitoring optical coherence tomography according to the above aspect of the present invention, preferably, the first communication module performs communication according to any one of Wireless LAN, Bluetooth, zigbee, and optical communication methods.

In the microscope for monitoring optical coherence tomography according to the above aspect of the present invention, preferably, the display unit is attached to a predefined area of the front of the ocular lens or is disposed in parallel to the ocular lens.

In the microscope for monitoring optical coherence tomography according to the above aspect of the present invention, preferably, the display unit has a smaller size than the ocular lens.

According to another aspect of the present invention, there is provided an optical coherence tomography image providing apparatus, including: a light source which generates light having a predefined wavelength; an optical coupler which divides the light into first and second light beams to apply the first and second light beams to a reference mirror and a sample, respectively, and couples a reference beam and a signal beam which are reflected from the reference mirror and the sample, respectively; a reference path unit which guides a path so as for the first light beam to be applied to the reference mirror and provide the reference beam reflected from the reference mirror to the optical coupler; a sample path unit which guides a path so as for the second light beam to be applied to the same position of the sample as the light of the microscope and provide the signal beam reflected from the sample to the optical coupler. a photodetector which detects an interference signal of the reference beam and the signal beam which are coupled by the optical coupler; a signal processing unit which generates an OCT image by processing the interference signal detected by the photodetector; and an image output unit which outputs the OCT image generated by the signal processing unit to the beam splitter of the microscope, wherein the optical coherence tomography image providing apparatus is combined with the microscope to generate the OCT image of the same position of the sample as the surface image of the microscope and provides the OCT image so as for the OCT image and the surface image of the sample to be simultaneously output.

In the optical coherence tomography image providing apparatus according to the above aspect of the present invention, the sample path unit is configured to include a dichroic mirror which is disposed between the first objective lens and the sample mount of the microscope to reflect the second light beam having a predefined wavelength and to transmit light having other wavelengths, and the dichroic mirror guides a path so as for the second light beam to be applied to the same position of the sample as light of the microscope.

In addition, the sample path unit is configured to further include: a collimator which collimates the second light beam; an optical path changing unit which is disposed on the propagation path of the collimated second light beam to propagate the second light beam to the dichroic mirror and to provide the signal beam reflected again from the sample to the collimator and is driven to scan a surface of the sample; and a second objective lens which is disposed between the dichroic mirror and the optical path changing unit to allow the second light beam output from the optical path changing unit to be focused and to provide the second light beam to the dichroic mirror.

In the present invention, preferably, the optical path changing unit of the sample path unit is configured with any one of a Galvano scanner, and a polygon mirror, and an X-Y scanner.

In the optical coherence tomography image providing apparatus according to the above aspect of the present invention, preferably, the image output unit is configured with a beam projector.

In the optical coherence tomography image providing apparatus according to the above aspect of the present invention, preferably, in the case of a binocular microscope where the microscope image providing unit has two beam splitters and two ocular lenses, the optical coherence tomography image providing apparatus provides the OCT image to at least one or more of the two beam splitters.

### [Advantageous Effects]

The microscope for monitoring optical coherence tomography according to the present invention has an advantageous effect in that light is illuminated on the same position of the sample as the microscope image providing unit through the sample path unit of the OCT image providing unit, so that the surface image and the OCT image of the same position of the sample can be simultaneously provided. Therefore, in the case where the microscope for monitoring optical coherence tomography according to the present invention is used during surgery, a surgeon can identify tomography information as well as the surface of a diseased part, so that it is possible to ensure safety and accuracy of the surgery.

The OCT image providing unit of the microscope for monitoring optical coherence tomography according to the present invention can be configured independently of the microscope image providing unit. Therefore, the optical coherence tomography image providing apparatus according to the present invention is configured to provide the OCT image to an existing microscope, so that the optical coherence tomography image providing apparatus has an advantage in that additional cost or time for modifying the microscope is not consumed.

In addition, the microscope for monitoring optical coherence tomography according to the present invention is configured so as to use the OCT for surgery, so that the microscope for monitoring optical coherence tomography is determined to contribute to widening the application fields of the OCT which was mainly used for diagnosis field and increasing the demand in the market.

In addition, since the microscope for monitoring optical coherence tomography according to the present invention can output various types of information such as surgical information as well as the OCT image by using the display unit installed in the microscope, the surface image and the OCT image of the sample, and information required for surgery can be provided to a surgeon through the ocular lens of the surgical microscope without turning his/her eyes during surgery.

In addition, in the microscope for monitoring optical coherence tomography according to the present invention, the display unit is separately installed inside the microscope, and thus, the path of the surface image of the microscope is not blocked, so that a vivid surface image and a high-quality OCT image can be provided.

### [Brief Description of Drawings]

Fig. 1 is a schematic diagram illustrating a structure of the microscope for monitoring optical coherence tomography according to a first embodiment of the present invention.
Fig. 2 is a schematic diagram illustrating a basic configuration of an OCT unit according to the first embodiment of the present invention.
Fig. 3 is a diagram illustrating a structure of a sample path unit of an OCT image providing unit according to the first embodiment of the present invention.
Fig. 4 is a diagram illustrating pictures of image in ocular lenses in the case were an OCT image is output in the microscope for monitoring optical coherence tomography according to a first embodiment of the present invention.
Fig. 5 is a diagram illustrating pictures of image in ocular lenses in the case were no OCT image is output in the microscope for monitoring optical coherence tomography according to the first embodiment of the present invention.
Fig. 6 is a schematic diagram illustrating a structure of the microscope for monitoring optical coherence tomography, according to the second embodiment of the present invention.
Fig. 7 is a schematic diagram illustrating a basic configuration of an OCT unit according to the second embodiment of the present invention.
Fig. 8 is a diagram illustrating a structure of a sample path unit of an OCT image providing unit according to the second embodiment of the present invention.
Fig. 9 is a diagram illustrating a picture of a microscope image in the case where an OCT image is output from a display unit in the microscope for monitoring optical coherence tomography according to the second embodiment of the present invention.

### [Best Modes for Carrying Out the Invention]

Hereinafter, a structure and a principle of operation of a microscope for monitoring optical coherence tomography according to the first embodiment of the present invention will be described in detail with reference to the attached drawings.

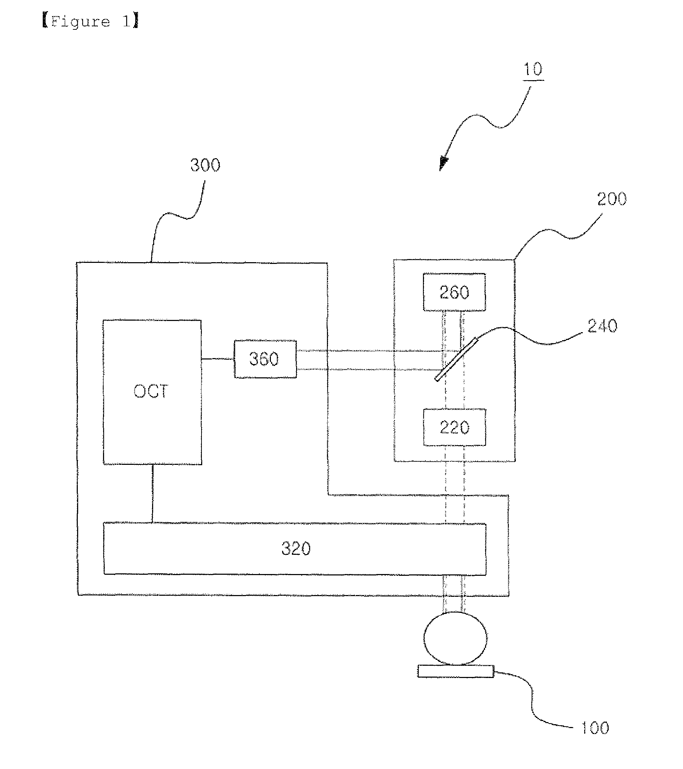

Fig. 1 is a schematic diagram illustrating a structure of the microscope for monitoring optical coherence tomography according to the first embodiment of the present invention. Referring to Fig. 1, the microscope for monitoring optical coherence tomography 10 according to the first embodiment of the present invention is configured to include a sample mount 100, a microscope image providing unit 200, and an optical coherent tomography (OCT) image providing unit 300. A sample is mounted on the sample mount 100, and in the present invention, a sample of a biological tissue such as an eyeball is used.

The microscope image providing unit 200 is configured to include a first objective lens 220, a beam splitter 240, and an ocular lens 260 which are sequentially disposed from the front of the sample mount 100 and provides a surface image of the sample by using first light generated from a first light source (not shown). The microscope image providing unit 200 has the same structure as that of a microscope of the related art, and particularly, has the structure where a beam splitter is disposed between an object lens and an ocular lens to output a surface image of a sample to an external camera as well as to the ocular lens. Therefore, besides the OCT image providing unit included in the microscope for monitoring optical coherence tomography according to the present invention, the later-described OCT image providing unit 300 may be a separate independent apparatus having a structure capable of being coupled with an existing microscope.

The OCT image providing unit 300 divides second light generated by a second light source into first and second light beams to apply the first and second light beams to a reference mirror and the sample, respectively, guides a path so as for the second light beam to be applied to the same position of the sample as the first light, generates an OCT image by using interference signals of a reference beam and a signal beam which are reflected from the reference mirror and the sample, respectively, and after that, provides the OCT image to a beam splitter 124 of the microscope image providing unit 200.

The OCT image providing unit of the microscope for monitoring optical coherence tomography having the above-described structure according to the present invention generates the OCT image of the same position as the surface image of the sample to provide the OCT image to the beam splitter of the microscope image providing unit, and in the case where the OCT image is provided through the beam splitter from the OCT image providing unit, the microscope image providing unit simultaneously outputs the surface image and the OCT image of the sample to the ocular lens.

In other words, since the path where the OCT image is generated and provided by the OCT image providing unit is independent of the path where the surface image is generated and provided by the microscope image providing unit, the microscope for monitoring optical coherence tomography can allow the surface image of the sample to be viewed in the same manner as the existing microscope and can provide the OCT image to overlap with the surface image according to user's selection.

Hereinafter, the aforementioned OCT image providing unit 300 will be described in detail.

Referring to Fig. 1, the OCT image providing unit 300 of the microscope for monitoring optical coherence tomography according to the present invention is configured to include an OCT unit, a sample path unit 320, and an image output unit 380.

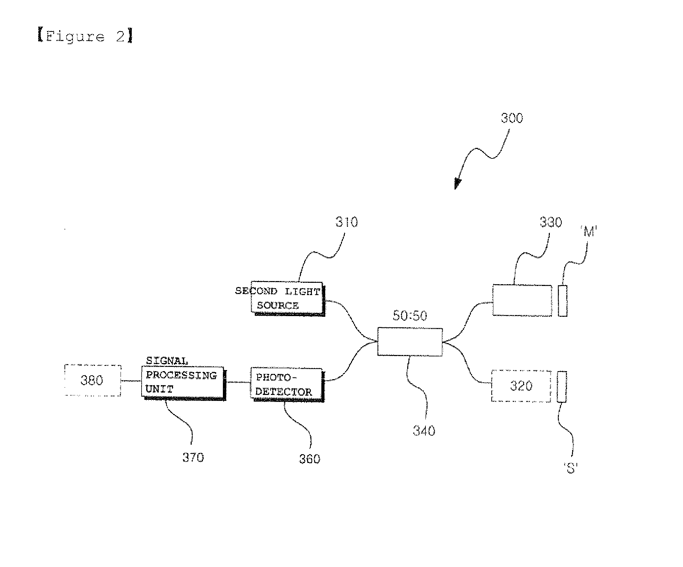

In the present invention, the OCT unit has the same basic configuration as that of an existing optical tomography imaging apparatus. Fig. 2 is a schematic diagram illustrating a basic configuration of the OCT unit according to the present invention. Referring to Fig. 2, the OCT unit is configured to include a second light source 310, an optical coupler 340, a reference path unit 330, a photodetector 360, and a signal processing unit 370. In the present invention, the OCT unit is organizationally connected through the sample path unit 320 and the image output unit 380 to the above-described microscope image providing unit 200.

The second light source 310 generates second light having a predefined wavelength. In the present invention, the wavelength of the second light source 310 is predefined, so that the second light source 310 is separated from the first light of the first light source of the microscope image providing unit 200 to allow the second light to propagate in an independent path.

Preferably, the second light source is a light source having a near infrared (IR) wavelength band, and in the present invention, an 850 nm light source is used.

The optical coupler 340 divides the second light into first and second light beams to apply the first and second light beams to a reference mirror 'M' and a sample 'S', respectively, and couples a reference beam and a signal beam which are reflected from the reference mirror and the sample, respectively.

The reference path unit 330 guides a path so as for the first light beam to be applied to the reference mirror 'M' and provides the reference beam reflected from the reference mirror to the optical coupler. As the reference path unit 330, any structure of guiding the path of the first light beam to the reference mirror can be used, and in general, a collimation lens is used.

The second light beam applied from the optical couple 340 to the sample is applied through the sample path unit 320 to the same position of the sample as the first light of the microscope image providing unit 200.

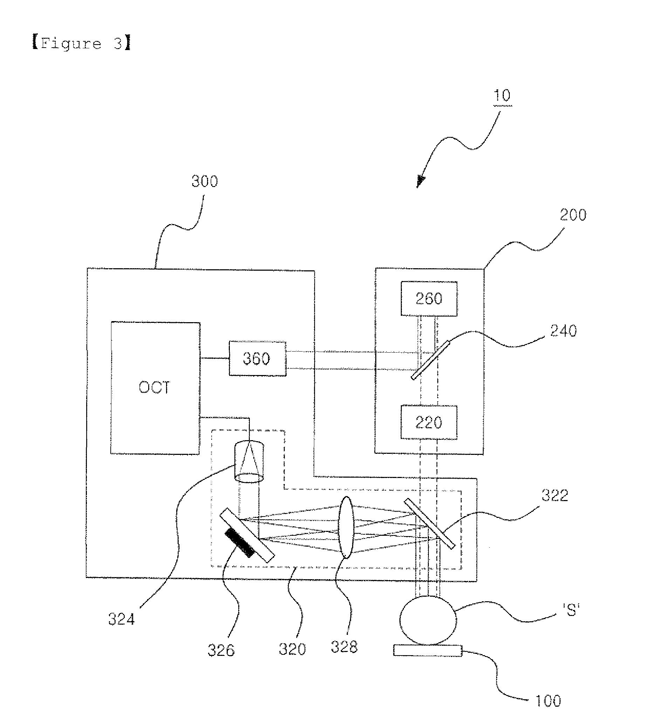

Fig. 3 is a diagram illustrating a structure of the sample path unit of the OCT image providing unit according to the present invention. Referring to Fig. 3, the sample path unit 320 is configured to include a dichroic mirror 322, a collimator 324, an optical path changing unit 326, and a second objective lens 328.

The collimator 324 collimates the second light beam. Since the second light beam is provided from the second light source is approximate to a point light source, the collimator 324 spreads the second light beam for collimation and uses the collimation lens to collimate the spread second light beam into a light beam having a uniform size.

The collimated second light beam propagates to the optical path changing unit 326.

The optical path changing unit 326 is disposed on the propagation path of the collimated second light beam to propagate the second light beam to the dichroic mirror and to provide the signal beam, which is reflected again from the sample to be reflected through the dichroic mirror, to the collimator again. The optical path changing unit 326 is driven to scan the surface of the sample so as to acquire the OCT image, and a Galvano scanner, a polygon mirror, an X-Y scanner, and the like may be used. In the present invention, the Galvano scanner is used as an example, and preferably, the Galvano scanner is a 2D-Galvano scanner to acquire a two-dimensional OCT image.

The second light beam provided from the optical path changing unit 326 is focused through the second objective lens 328 and propagates to the dichroic mirror 322. The second objective lens 328 provides the signal beam reflected again by the sample to the optical path changing unit 326. The dichroic mirror 322 is disposed between the first objective lens 220 of the microscope image providing unit and the sample mount 100. In general, the dichroic mirror has a property of reflecting light having a specific wavelength and transmitting light having other wavelengths. In the present invention, the second light beam having a predefined wavelength which propagates from the second light source through the optical path changing unit is reflected to be provided to the sample. At this time, the propagation path of the reflected second light beam needs to be the same as the propagation path of the first light of the microscope image providing unit 200. In other words, the dichroic mirror 322 of the sample path unit 320 guides the propagation path of the second light beam to the same position of the sample as the first light for providing the surface image of the sample, so that the microscope for monitoring optical coherence tomography according to the present invention can provide the OCT image of the same position as the surface image.

Since the dichroic mirror 322 reflects light having a specific wavelength and transmits light having other wavelengths, the microscope image providing unit which uses the first light having a visible wavelength band can provide the surface image of the sample irrespective of the OCT image providing unit.

The sample path unit 320 having the above-described components according to the present invention propagates the second light beam divided by the optical coupler 340 sequentially through the collimator 324, the optical path changing unit 326, and the second objective lens 328 to the dichroic mirror 322 to provide the second light beam to the same position of the sample as the microscope image providing unit 200 and propagates the signal beam reflected again by the sample in the reverse order of the above-described components to provide the signal beam to the optical coupler 340 again.

The photodetector 360 detects an interference signal of the reference beam and the signal beam which are coupled by the optical coupler 340.

The signal processing unit 370 generates the OCT image by processing the interference signal detected by the photodetector.

The generated OCT image is output through the image output unit 360 to the beam splitter 240 of the microscope image providing unit 200. In the present invention, preferably, the image output unit 360 is configured with one of a beam projector, an LCD (Liquid Crystal Display), and an OLED (Organic Light-Emitting Display).

The microscope for monitoring optical coherence tomography having the above-described structure according to the present invention has an advantage in that the microscope for monitoring optical coherence tomography illuminates the same position of the sample as the microscope image providing unit with light through the sample path unit 320 of the OCT image providing unit 300, so that it is possible to output the surface image and the OCT image of the same position of the sample. Therefore, in the case where the microscope for monitoring optical coherence tomography according to the present invention is used during surgery, a surgeon can identify tomography information as well as the surface of a diseased part, so that it is possible to ensure safety and accuracy of the surgery.

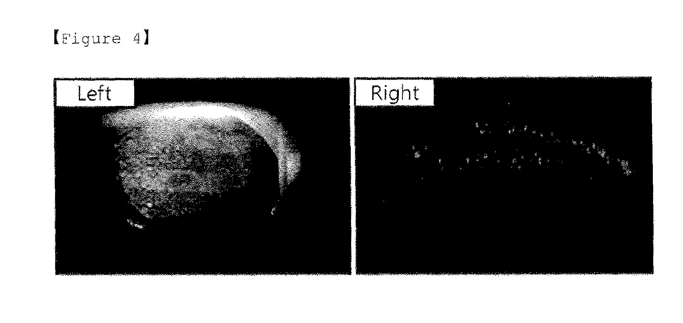

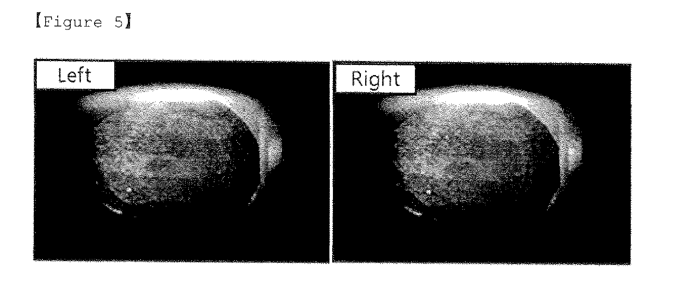

Fig. 4 is a diagram illustrating pictures of image in ocular lenses in the case were an OCT image is output in the microscope for monitoring optical coherence tomography according to the present invention, and Fig. 5 is a diagram illustrating pictures of image in ocular lenses in the case were no OCT image is output. Herein, the sample is an eyeball of a rabbit.

Referring to Figs. 4 and 5, the microscope is a binocular microscope having two ocular lenses, and in the present invention, it can be understood that the OCT image can be output through only one-side ocular lens. The binocular microscope is configured to include two beam splitters in order to output respective images to the two ocular lenses, and the above-described OCT image providing unit 300 can provide the OCT image to at least one or more of the two beam splitters. In Figs. 4 and 5, the OCT image is provided to the left ocular lens. Referring to Fig. 5, it can be understood that, in the case where no OCT image is provided, like the existing microscope, the only surface image is output.

On the other hand, the OCT image providing unit of the above-described microscope for monitoring optical coherence tomography may be configured independently of the microscope image providing unit. Namely, the microscope image providing unit may be configured with an existing surgical microscope, and the OCT image providing unit may be configured with an OCT image providing apparatus capable of being combined to the surgical microscope. In this case, the configuration of the OCT image providing apparatus is the same as that of the above-described OCT image providing unit. Therefore, the optical coherence tomography image providing apparatus according to the present invention is configured to be combined to an existing microscope to provide the OCT image, so that the optical coherence tomography image providing apparatus has an advantage in that additional cost or time for modifying the microscope is not consumed.

### [Embodiments]

Hereinafter, a structure and a principle of operation of a microscope for monitoring optical coherence tomography according to a second embodiment of the present invention will be described in detail with reference to the attached drawings.

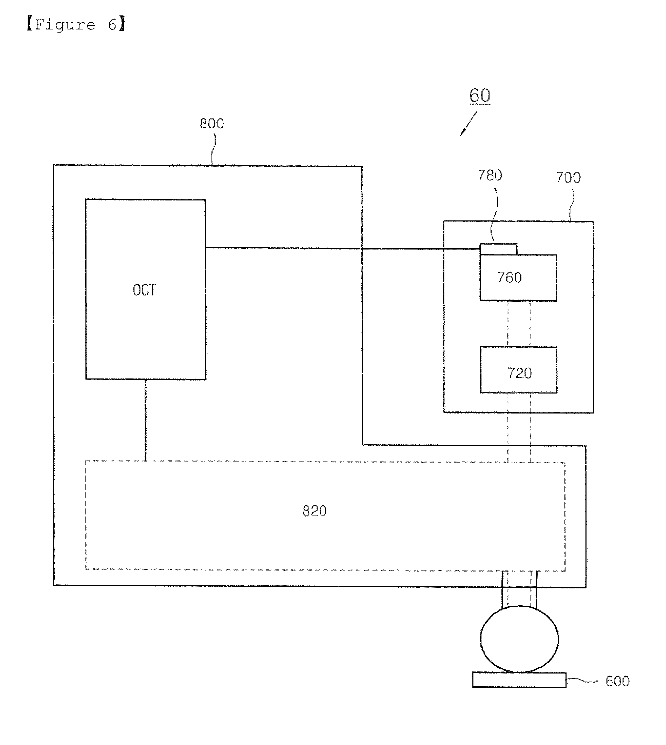

Fig. 6 is a schematic diagram illustrating a structure of the microscope for monitoring optical coherence tomography according to the second embodiment of the present invention. Referring to Fig. 6, the microscope for monitoring optical coherence tomography 60 according to the second embodiment of the present invention is configured to include a sample mount 600, a microscope image providing unit 700, and an optical coherent tomography (OCT) image providing unit 800. A sample is mounted on the sample mount 600, and in the present invention, a sample of a biological tissue such as an eyeball is used.

The microscope image providing unit 700 is configured to include a first objective lens 720 and an ocular lens 760 which are sequentially disposed from the front of the sample mount 600 and a display unit 780 which is installed in a predetermined area to output an image on a screen according to an image signal provided from an external system and outputs the image by using first light generated by a first light source (not shown) at the same time of providing the surface image of the sample. The microscope image providing unit 700 has the same structure as that of a microscope of the related art, and the display unit 780 is installed in a predetermined area in front of the ocular lens 760 or is disposed in parallel to the ocular lens.

Herein, the display unit 780 needs to have a smaller size than the ocular lens, and thus, when the display unit is attached to the front of the ocular lens, the display unit 780 needs not to block the output of the surface image of the sample provided to the ocular lens. Therefore, in the case where the display unit 780 is attached to the front of the ocular lens, preferably, the display unit is disposed in an area deviated by a predetermined distance from the center of the ocular lens, and more preferably, the display unit is disposed in parallel to the ocular lens.

In addition, the display unit 780 is configured to include a display panel which outputs an image on a screen according to an image signal and a first communication module which receives the image signal from an external portion. The display panel is configured with a subminiature display apparatus, and preferably, one of an LCD (Liquid Crystal Display), an OLED (Organic Light Emitting Diode), and an LCoS (Liquid Crystal on Silicon) is used.

These display apparatuses have so high resolution to provide vivid images.

The first communication module is a module for receiving the image signal from the external portion, and wired communication and wireless communication are available. In the case of wireless communication, preferably, the communication is performed according to any one of wireless LAN, Bluetooth, zigbee, and optical fiber communication methods.

The OCT image providing unit 800 divides second light generated by a second light source into first and second light beams to apply the first and second light beams to a reference mirror and the sample, respectively, guides a path so as for the second light beam to be applied to the same position of the sample as the first light, generates an OCT image by using interference signals of a reference beam and a signal beam which are reflected from the reference mirror and the sample, respectively, and after that, outputs an OCT image signal corresponding to the OCT image to the display unit of the microscope image providing unit.

The OCT image providing unit of the microscope for monitoring optical coherence tomography having the above-described structure according to the present invention generates the OCT image of the same position as the surface image of the sample and, after that, outputs the OCT image signal corresponding to the generated OCT image to the display unit of the microscope image providing unit, and in the case where the OCT image signal is provided through the display unit from the OCT image providing unit, the microscope image providing unit simultaneously outputs the surface image and the OCT image of the sample. In other words, since the path where the OCT image is generated and provided by the OCT image providing unit is independent of the path where the surface image is generated and provided by the microscope image providing unit, the microscope for monitoring optical coherence tomography can allow the surface image of the sample to be viewed in the same manner as the existing microscope and can simultaneously provide the surface image and the OCT image in the same view of the microscope through the display unit which is attached to a predefined area of the ocular lens or is disposed in parallel to the ocular lens.

Hereinafter, the above-described OCT image providing unit 800 will be described in detail.

Referring to Fig. 6, the OCT image providing unit 800 of the microscope for monitoring optical coherence tomography according to the present invention is configured to include an OCT unit and a sample path unit 820.

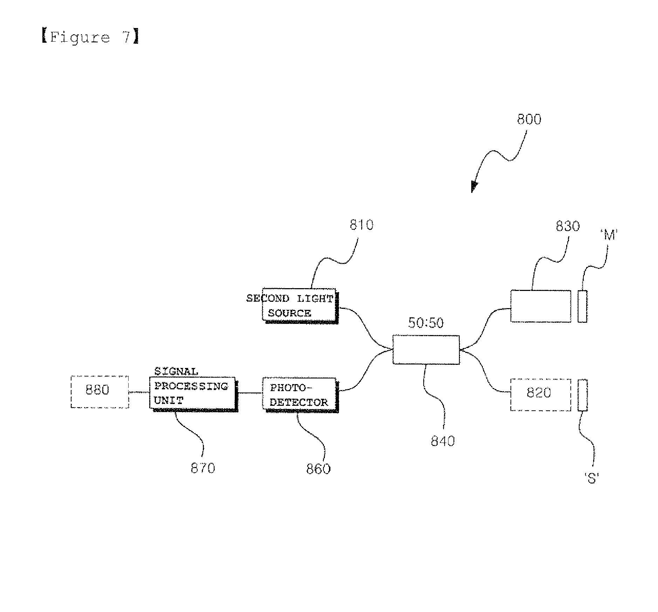

In the present invention, the OCT unit has the same basic configuration as that of an existing optical tomography imaging apparatus. Fig. 7 is a schematic diagram illustrating a basic configuration of the OCT unit according to the second embodiment of the present invention. Referring to Fig. 7, the OCT unit is configured to a second light source 810, an optical coupler 840, a reference path unit 830, a photodetector 860, a signal processing unit 870, and a second communication module (not shown). In the present invention, the OCT unit communicates with the display unit of the microscope image providing unit 700 through the second communication module. Preferably, the second communication module is selected according to the communication method of the first communication module.

The second light source 810 generates second light having a predefined wavelength. In the present invention, the wavelength of the second light source 810 is predefined, so that the second light source 810 is separated from the first light of the first light source of the microscope image providing unit 700 to allow the second light to propagate in an independent path.

Preferably, the second light source is a light source having a near infrared (IR) wavelength band, and in the present invention, an 850 nm light source is used.

The optical coupler 840 divides the second light into first and second light beams to apply the first and second light beams to a reference mirror 'M' and a sample 'S', respectively, and couples a reference beam and a signal beam which are reflected from the reference mirror and the sample, respectively.

The reference path unit 830 guides a path so as for the first light beam to be applied to the reference mirror 'M' and provides the reference beam reflected from the reference mirror to the optical coupler. As the reference path unit 830, any structure of guiding the path of the first light beam to the reference mirror can be used, and in general, a collimation lens is used.

The second light beam applied from the optical couple 840 to the sample is applied through the sample path unit 820 to the same position of the sample as the first light of the microscope image providing unit 200.

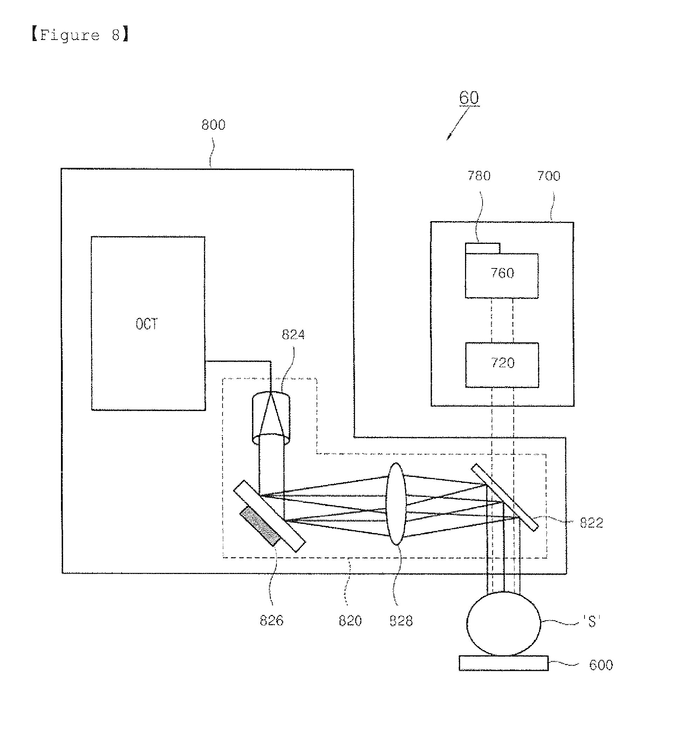

Fig. 8 is a diagram illustrating a structure of the sample path unit of the OCT image providing unit according to the present invention. Referring to Fig. 8, the sample path unit 820 is configured to include a dichroic mirror 822, a collimator 824, an optical path changing unit 826, and a second objective lens 828.

The collimator 824 collimates the second light beam. Since the second light beam is provided from the second light source is approximate to a point light source, the collimator 824 spreads the second light beam for collimation and uses the collimation lens to collimate the spread second light beam into a light beam having a uniform size.

The collimated second light beam propagates to the optical path changing unit 826.

The optical path changing unit 826 is disposed on the propagation path of the collimate second light beam to propagate the second light beam to the dichroic mirror and to provide the signal beam, which is reflected again from the sample to be reflected through the dichroic mirror, to the collimator again. The optical path changing unit 826 is driven to scan the surface of the sample so as to acquire the OCT image, and a Galvano scanner, a polygon mirror, an X-Y scanner, and the like may be used. In the present invention, the Galvano scanner is used as an example, and preferably, the Galvano scanner is a 2D-Galvano scanner to acquire a two-dimensional OCT image.

The second light beam provided from the optical path changing unit 826 is focused through the second objective lens 828 and propagates to the dichroic mirror 822. The second objective lens 828 provides the signal beam reflected again by the sample to the optical path changing unit 826. The dichroic mirror 822 is disposed between the first objective lens 220 of the microscope image providing unit and the sample mount 100. In general, the dichroic mirror has a property of reflecting light having a specific wavelength and transmitting light having other wavelengths. In the present invention, the second light beam having a predefined wavelength which propagates from the second light source through the optical path changing unit is reflected to be provided to the sample. At this time, the propagation path of the reflected second light beam needs to be the same as the propagation path of the first light of the microscope image providing unit 700. In other words, the dichroic mirror 822 of the sample path unit 820 guides the propagation path of the second light beam to the same position of the sample as the first light for providing the surface image of the sample, so that the microscope for monitoring optical coherence tomography according to the present invention can provide the OCT image of the same position as the surface image.

Since the dichroic mirror 822 reflects light having a specific wavelength and transmits light having other wavelengths, the microscope image providing unit which uses the first light having a visible wavelength band can provide the surface image of the sample irrespective of the OCT image providing unit.

The sample path unit 820 having the above-described components according to the present invention propagates the second light beam divided by the optical coupler 840 sequentially in through collimator 824, the optical path changing unit 826, and the second objective lens 828 to dichroic mirror 822 to provide the second light beam to the same position of the sample as the microscope image providing unit 700 and propagates the signal beam reflected again by the sample in the reverse order of the above-described components to provide the signal beam to the optical coupler 840 again.

The photodetector 860 detects an interference signal of the reference beam and the signal beam which are coupled by the optical coupler 840.

The signal processing unit 870 generates the OCT image by processing the interference signal detected by the photodetector and, after that, outputs the image signal corresponding to the generated OCT image. The output image signal is provided through the second communication module to the display unit of the microscope image providing unit, and more specifically, the output image signal is provided through communication with the first communication module of the display unit.

The microscope for monitoring optical coherence tomography having the above-described structure according to the present invention has an advantage in that the microscope for monitoring optical coherence tomography illuminates the same position of the sample as the microscope image providing unit with light through the sample path unit 820 of the OCT image providing unit 800, so that it is possible to output the surface image and the OCT image of the same position of the sample. In addition, since the microscope, for monitoring optical coherence tomography according to the present invention can output various types of information such as surgical information as well as the OCT image by using the display unit installed in the microscope, the surface image and the OCT image of the sample, and information required for surgery can be provided to a surgeon through the ocular lens of the surgical microscope without turning his/her eyes during surgery.

In addition, in the case where a surface image and an OCT image are provided so as to overlap with each other through a beam splitter added to a microscope, contrast of the surface image of the microscope may be decreased due to the above added optical system. Therefore, in the microscope for monitoring optical coherence tomography 60 according to the present invention, the display unit 780 is separately installed inside the microscope, and thus, the path of the surface image of the microscope is not blocked, so that a vivid surface image surface image and a high-quality OCT image can be provided.

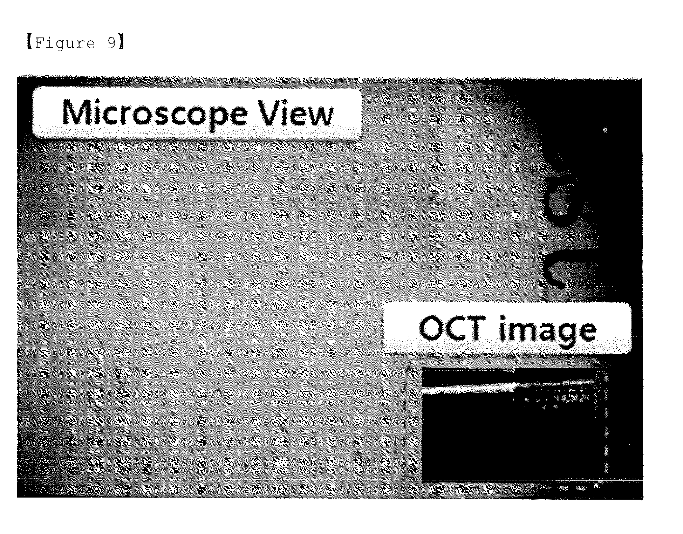

Fig. 9 is a diagram illustrating a picture of a microscope image in the microscope for monitoring optical coherence tomography according to the second embodiment in the case where the OCT image is output through the display unit. Referring to Fig. 9, it can understood that the images output through the microscope are the surface image and the OCT image of the sample shown on the display unit disposed in a predetermined area of the ocular lens. Since the display unit having a smaller size than the ocular lens is attached to one side of the ocular lens, the display unit does not greatly block the view of the surface image of the microscope image providing unit, so that it is possible to effectively provide the surface image and the OCT image. While the present invention has been particularly shown and described with reference to exemplary embodiments thereof, it will be understood by those skilled in the art that various changes in form and details may be made therein without departing from the spirit and scope of the invention as defined by the appended claims. The exemplary embodiments should be considered in descriptive sense only and not for purposes of limitation. Therefore, the scope of the invention is defined not by the detailed description of the invention but by the appended claims, and all differences within the scope will be construed as being included in the present invention.

### [Industrial Applicability]

A microscope for monitoring optical coherence tomography according to the present invention can be applied to all the fields using microscopes, and more particularly, can be applied to a surgical microscope required for accurate diagnosis and surgery of diseased parts.

## Claims

1. A microscope for monitoring optical coherence tomography, comprising:
a sample mount which a sample is mounted on;
a microscope image providing unit which is configured to include a first objective lens and an ocular lens which are sequentially disposed from the front of the sample mount and
provides a surface image of the sample by using first light generated by a first light source; and
an OCT image providing unit which divides second light generated by a second light source into first and second light beams to apply the first and second light beams to a reference mirror and the sample, respectively, guides a path so as for the second light beam to be applied to the same position of the sample as the first light, generates an optical coherent tomography (OCT) image by using interference signals of a reference beam and a signal beam which are reflected from the reference mirror and the sample, respectively, and after that, provides the OCT image or an OCT image signal corresponding to the OCT image to the microscope image providing unit,
wherein the OCT image providing unit generates the OCT image of the same position as the surface image of the sample and provides the OCT image to the microscope image providing unit.

2. The microscope for monitoring optical coherence tomography according to claim 1, wherein the microscope image providing unit is configured to further include a beam splitter between the first objective lens and the ocular lens, and in the case where the OCT image is provided from the OCT image providing unit to the beam splitter, the microscope image providing unit simultaneously outputs the surface image and the OCT image of the sample to the ocular lens.

3. The microscope for monitoring optical coherence tomography according to claim 2, wherein the OCT image providing unit is configured to include:
a second light source which generates the second light having a predefined wavelength;
an optical coupler which divides the second light into the first and second light beams to apply the first and second light beams to the reference mirror and the sample, respectively, and couples a reference beam and a signal beam which are reflected from the reference mirror and the sample, respectively;
a reference path unit which guides a path so as for the first light beam to be applied to the reference mirror and
provides the reference beam reflected from the reference mirror to the optical coupler;
a sample path unit which guides a path so as for the second light beam to be applied to the same position of the sample as the first light and provides the signal beam reflected from the sample to the optical coupler;
a photodetector which detects an interference signal of the reference beam and the signal beam which are coupled by the optical coupler;
a signal processing unit which generates the OCT image by processing the interference signal detected by the photodetector; and
an image output unit which outputs the OCT image generated by the signal processing unit to the beam splitter of the microscope image providing unit.

4. The microscope for monitoring optical coherence tomography according to claim 1, wherein the microscope image providing unit is configured to further include a display unit which is installed in a predetermined area to output an image on a screen according to an image signal provided from an external system, and in the case where an OCT image signal corresponding to the OCT image is provided from the OCT image providing unit to the display unit, the microscope image providing unit simultaneously outputs the surface image and the OCT image of the sample.

5. The microscope for monitoring optical coherence tomography according to claim 4, wherein the OCT image providing unit is configured to include:
a second light source which generates the second light having a predefined wavelength;
an optical coupler which divides the second light into the first and second light beams to apply the first and second light beams to the reference mirror and the sample, respectively, and couples a reference beam and a signal beam which are reflected from the reference mirror and the sample, respectively;
a reference path unit which guides a path so as for the first light beam to be applied to the reference mirror and provides the reference beam reflected from the reference mirror to the optical coupler;
a sample path unit which guides a path so as for the second light beam to be applied to the same position of the sample as the first light and provides the signal beam reflected from the sample to the optical coupler;
a photodetector which detects an interference signal of the reference beam and the signal beam which are coupled by the optical coupler;
a signal processing unit which generates the OCT image by processing the interference signal detected by the photodetector and outputs the OCT image signal corresponding to the generated OCT image; and
a second communication module which provides the output OCT image signal to the display unit of the microscope image providing unit.

6. The microscope for monitoring optical coherence tomography according to claim 3 or 5,
wherein the sample path unit is configured to include a dichroic mirror which is disposed between the first objective lens and the sample mount to reflect the second light beam having a predefined wavelength and to transmit light having other wavelengths, and
wherein the dichroic mirror guides a path so as for the second light beam to be applied to the same position of the sample as the first light.

7. The microscope for monitoring optical coherence tomography according to claim 6, wherein the sample path unit is configured to further include:
a collimator which collimates the second light beam;
an optical path changing unit which is disposed on the propagation path of the collimated second light beam to propagate the second light beam to the dichroic mirror and to provide the signal beam reflected again from the sample to the collimator and is driven to scan a surface of the sample; and
a second objective lens which is disposed between the dichroic mirror and the optical path changing unit to allow the second light beam output from the optical path changing unit to be focused and to provide the focused second light beam to the dichroic mirror.

8. The microscope for monitoring optical coherence tomography according to claim 7, wherein the optical path changing unit is configured with any one of a Galvano scanner, and a polygon mirror, and an X-Y scanner.

9. The microscope for monitoring optical coherence tomography according to claim 3, wherein the image output unit is configured with any one of a beam projector, an LCD (Liquid Crystal Display), and an OLED (Organic Light-Emitting Display).

10. The microscope for monitoring optical coherence tomography according to claim 2, wherein, in the case of a binocular microscope where the microscope image providing unit has two beam splitters and two ocular lenses, the OCT image providing unit provides the OCT image to at least one or more of the two beam splitters.

11. The microscope for monitoring optical coherence tomography according to claim 4, wherein the display unit is configured to include:
a display panel which outputs the image on the screen according to the image signal; and
a first communication module which receives the image signal from the external portion.

12. The microscope for monitoring optical coherence tomography according to claim 11, wherein the display panel is configured with any one of an LCD (Liquid Crystal Display), an OLED (Organic Light Emitting Diodes), and an LCoS (Liquid Crystal on Silicon).

13. The microscope for monitoring optical coherence tomography according to claim 11, wherein the first communication module performs communication according to any one of Wireless LAN, Bluetooth, zigbee, and optical communication methods.

14. The microscope for monitoring optical coherence tomography according to claim 4, wherein the display unit is attached to a predefined area of the front of the ocular lens or is disposed in parallel to the ocular lens.

15. The microscope for monitoring optical coherence tomography according to claim 4, wherein the display unit has a smaller size than the ocular lens.

16. An optical coherence tomography image providing apparatus, comprising:
a light source which generates light having a predefined wavelength;
an optical coupler which divides the light into first and second light beams to apply the first and second light beams to a reference mirror and a sample, respectively, and couples a reference beam and a signal beam which are reflected from the reference mirror and the sample, respectively;
a reference path unit which guides a path so as for the first light beam to be applied to the reference mirror and provide the reference beam reflected from the reference mirror to the optical coupler;
a sample path unit which guides a path so as for the second light beam to be applied to the same position of the sample as the light of the microscope and provide the signal beam reflected from the sample to the optical coupler;
a photodetector which detects an interference signal of the reference beam and the signal beam which are coupled by the optical coupler;
a signal processing unit which generates an OCT image by processing the interference signal detected by the photodetector; and
an image output unit which outputs the OCT image generated by the signal processing unit to the beam splitter of the microscope,
wherein the optical coherence tomography image providing apparatus is combined with the microscope to generate the OCT image of the same position of the sample as the surface image of the microscope and provides the OCT image so as for the OCT image and the surface image of the sample to be simultaneously output.

17. The optical coherence tomography image providing apparatus according to claim 16,
wherein the sample path unit is configured to include a dichroic mirror which is disposed between the first objective lens and the sample mount of the microscope to reflect the second light beam having a predefined wavelength and to transmit light having other wavelengths, and
wherein the dichroic mirror guides a path so as for the second light beam to be applied to the same position of the sample as light of the microscope.

18. The optical coherence tomography image providing apparatus according to claim 17, wherein the sample path unit is configured to further include:
a collimator which collimates the second light beam;
an optical path changing unit which is disposed on the propagation path of the collimated second light beam to propagate the second light beam to the dichroic mirror and to provide the signal beam reflected again from the sample to the collimator and is driven to scan a surface of the sample; and
a second objective lens which is disposed between the dichroic mirror and the optical path changing unit to allow the second light beam output from the optical path changing unit to be focused and to provide the second light beam to the dichroic mirror.

19. The optical coherence tomography image providing apparatus according to claim 18, wherein the optical path changing unit is configured with any one of a Galvano scanner, and a polygon mirror, and an X-Y scanner.

20. The optical coherence tomography image providing apparatus according to claim 16, wherein the image output unit is configured with any one of a beam projector, an LCD (Liquid Crystal Display), and an OLED (Organic Light-Emitting Display).

21. The optical coherence tomography image providing apparatus according to claim 16, wherein, in the case of a binocular microscope where the microscope image providing unit has two beam splitters and two ocular lenses, the optical coherence tomography image providing apparatus provides the OCT image to at least one or more of the two beam splitters.
